# EUROPÄISCHE PATENTANMELDUNG

(11) **EP 0 542 391 A1**
(43) Veröffentlichungstag der Anmeldung: **19.05.1993**
(21) Anmeldenummer: 92250338.8
(22) Anmeldetag: 13.11.1992
(51) Int. Cl.: G01N 33/00, G01N 1/28

(54) **Verfahren und Vorrichtung zur Konzentrationsmessung eines schweren Gases niedriger Konzentration in einem Gasstrom**

(30) Priorität: 15.11.1991 DE 4138177
(71) Anmelder: BEUTLER MASCHINENBAU- UND VERTRIEBSGESELLSCHAFT INHABER WOLFGANG BEUTLER, D-23758 Göhl (DE)
(72) Erfinder: Beutler, Wolfgang, W-2440 Göhl (DE)
(74) Vertreter: Jander, Dieter, Dipl.-Ing.

(57) **Zusammenfassung**

Die Erfindung bezieht sich auf ein Verfahren zur Konzentrationsmessung eines schwereren Gases niedriger Konzentration in einem Gasstrom, bestehend aus diesem Gas und einem leichteren Gas hoher Konzentration mit einem Gasdetektor. Die Erfindung besteht darin, daß man den Gasstrom in Rotation versetzt, diesen in einen ersten, an dem schwereren Gas angereicherten Teil und in einen zweiten, an dem schwereren Gas verarmten Teil auftrennt, den ersten Teil der Meßstelle zuführt und die beiden Teile anschließend wieder zusammenführt. Auf diese Weise können sehr niedrige Konzentrationen des schwereren Gases in dem Gasgemisch gemessen werden.

## Beschreibung

Die Erfindung bezieht sich auf ein Verfahren zur Konzentrationsmessung eines schwereren Gases niedriger Konzentration in einem Gasstrom, bestehend aus diesem Gas und einem leichteren Gas hoher Konzentration mit einem Gasdetektor.

Als schwereres Gas kommt z.B. ein Kältemittel wie R 123, als leichteres Gas z.B. Luft in Frage.

Die bekannten Verfahren dieser Art lassen die Erfassung dieser Gase bis zu etwa 25ppm zu. Kleinere Konzentrationen sind wegen gewisser Störeinflüsse nicht erfaßbar.

Der Erfindung liegt die Aufgabe zugrunde, das eingangs erwähnte Verfahren derart auszuführen, daß kleinere Konzentrationen erfaßt werden können.

Diese Aufgabe wird erfindungsgemäß dadurch gelöst, daß man den Gasstrom in Rotation versetzt, diesen in einen ersten, an dem schwereren Gas angereicherten Teil und in einen zweiten, an dem schwereren Gas verarmten Teil auftrennt, den ersten Teil der Meßstelle zuführt und die beiden Teile anschließend wieder zusammenführt.

Auf diesem Wege ist es auf einfache Art und Weise möglich, niedrige Konzentrationen, z.B. von 10 ppm, des schwereren Gases zu erfassen.

Es sei erwähnt, daß es bereits bekannt ist, einen Gasstrom in Rotation zu versetzen und diesen in einen ersten, an einem schwereren Gas angereicherten Teil und in einen zweiten, an dem schwereren Gas verarmten Teil aufzutrennen, um auf diese Weise das schwerere und das leichtere Gas voneinander zu trennen (DE-OS 300 063).

Eine Weiterentwicklung der Erfindung besteht darin, daß der erste Teil durch eine Pumpe angetrieben wird und der zweite Teil an der Zusammenführungsstelle der beiden Teile durch Saugwirkung mitgerissen wird.

Eine andere Weiterentwicklung besteht darin, daß der erste Teil durch eine erste Pumpe und der Gasstrom nach der Zusammenführung durch eine zweite Pumpe angetrieben wird. Diese Lösung ist besonders dann interessant, wenn der Gasstrom nicht sogleich ins Freie, sondern in eine weitere Leitung geführt wird.

Die Erfindung bezieht sich auch auf eine Vorrichtung zur Durchführung des erwähnten Verfahrens. Diese ist erfindungsgemäß gekennzeichnet durch eine Leitungsführung, insbesondere Leitflächen, auf der Gaseinströmseite, die den Gasstrom in Rotation versetzt (versetzen), eine sich daran anschliessende Leitungsverzweigung, die den Gasstrom in die beiden Teile aufteilt, und eine Leitungszusammenführung, die die beiden Teile wieder vereinigt, wobei der Gasdetektor in die Zweigleitung ragt, die den ersten Teil führt, eine Gasförderpumpe sich in dieser Zweigleitung befindet und die Leitungszusammenführung nach Art einer Strahlpumpe ausgebildet ist, bei der der zweite Teil von dem ersten Teil mitgerissen wird.

Weiterhin wird vorgeschlagen, daß die Pumpe in Strömungsrichtung vor dem Gasdetektor liegt.

Sodann ist es zweckmäßig, wenn eine zylindrische erste Kammer an einem Ende einen vorzugsweise peripherisch einmündenden Gaseingang mit sich daran anschließenden Leitflächen und ein zentrales Gasausgangsrohr aufweist, daß sich am anderen Ende der ersten Kammer einerseits ein außen befindlicher Gasausgang und andererseits ein zentrales Leitungsstück befindet, wobei eine weitere Leitung, in der die Pumpe und der Detektor liegen, den Gasausgang mit dem Leitungsstück verbindet und wobei das Leitungsstück eingangsseitig aus einem koaxialen Doppelrohrstück besteht, von dem der innere Teil mit der weiteren Leitung verbunden ist und in dem äußeren Teil des Leitungsstückes endet und der äußere Teil sich eingangsseitig in die erste Kammer öffnet und ausgangsseitig in das Gasausgangsrohr übergeht.

Weiterhin wird vorgeschlagen, daß die weitere Leitung ausgangsseitig eine zweite Kammer bildet, in der sich der Gasdetektor an einer Stelle befindet, wo möglichst wenig Gasbewegung herrscht.

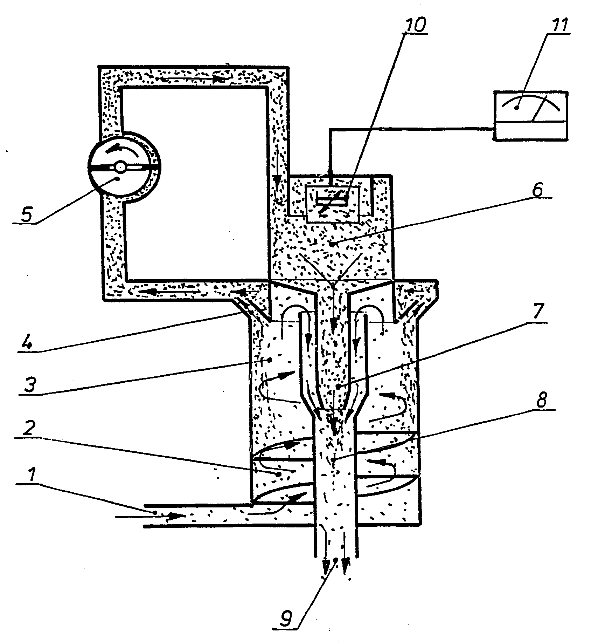

Einzelheiten der Erfindung ergeben sich aus der Zeichnung, die eine Vorrichtung zur Durchführung des erfindungsgemäßen Verfahrens zeigt.

Darin ist (sind) mit 1 ein Gaseingangsrohr, mit 2 Leitflächen, mit 3 eine erste Kammer, mit 4 ein Gasausgang, mit 5 eine Pumpe, mit 6 eine zweite Kammer, mit 7 ein Leitungsstück, mit 8 ein Gasausgangsrohr, mit 9 dessen Ausgangsmündung, mit 10 ein Gasdetektor und mit 11 ein mit dem Gasdetektor 10 verbundenes Anzeigegerät bezeichnet.

In das Gaseinströmrohr strömt die Umgebungsluft, die mit einem schwereren Gas niedriger Konzentration angereichert ist, ein. Die Leitflächen 2 sorgen dafür, daß es in der Kammer 3 in Rotation gerät. Diese Rotation hat zur Folge, daß das schwere Gas außen in der Kammer sich anreichert dergestalt, daß durch die Gasöffnung 4 in die Leitung, in der sich die Pumpe 5 befindet, ein Gasgemisch strömt, das an dem schweren Gas angereichert ist. Dieses strömt dann in die zweite Kammer 6 und mittels des Gasdetektors 10 kann problemlos das schwerere Gas erfaßt werden. Die Komponente des Luftgasgemisches, die hinsichtlich des schwereren Gases verarmt ist, strömt unmittelbar in das Leitungsstück 7,8. Am Ende dieses Leitungsstückes vereinigen sich die beiden Komponenten wieder, wobei der innere Gasstrom, der durch die Pumpe 5 angetrieben wird, den äußeren Gasstrom mitreißt. Aus der Öffnung 9 strömt dann wieder das Gasgemisch mit seinen ursprünglichen Konzentrationsverhältnissen aus.

Die Anzeige, die man erhält, ist für stets gleiche Verhältnisse (gleiche Pumpenleistung, gleiche Konzentration usw.) stets gleich. Ist das Instrument vorher geeicht worden, kann man an ihm die Konzentration des schwereren Gases außerhalb der erfindungsgemäßen Vorrichtung ablesen.

## Patentansprüche

1. Verfahren zur Konzentrationsmessung eines schwereren Gases niedriger Konzentration in einem Gasstrom, bestehend aus diesem Gas und einem leichteren Gas hoher Konzentration mit einem Gasdetektor, **dadurch gekennzeichnet**, daß man
a) den Gasstrom in Rotation versetzt,
b) diesen in einen ersten, an dem schwereren Gas angereicherten Teil und in einen zweiten, an dem schwereren Gas verarmten Teil auftrennt,
c) den ersten Teil der Meßstelle zuführt und
d) die beiden Teile anschließend wieder zusammenführt.

2. Verfahren nach Anspruch 1, **dadurch gekennzeichnet**, daß der erste Teil durch eine erste Pumpe (5) angetrieben wird und der zweite Teil an der Zusammenführungsstelle (7,8) der beiden Teile durch Saugwirkung mitgerissen wird.

3. Verfahren nach Anspruch 1 oder 2, **dadurch gekennzeichnet,** daß der erste Teil durch eine erste Pumpe (5) und der Gasstrom nach der Zusammenführung durch eine zweite Pumpe angetrieben wird.

4. Vorrichtung zur Durchführung des Verfahrens nach Anspruch 1 oder 2, **gekennzeichnet durch** eine Leitungsführung, insbesondere Leitflächen (2), auf der Gasstromeingangsseite, die den Gasstrom in Rotation versetzt (versetzen), eine sich daran anschließende Leitungsverzweigung (4), die den Gasstrom in die beiden Teile aufteilt, und eine Leitungszusammenführung (7,8), die die beiden Teile wieder vereinigt, wobei der Gasdetektor (10) in die Zweigleitung ragt, die den ersten Teil führt, eine erste Pumpe (5) sich in dieser Zweigleitung befindet und vorzugsweise die Leitungszusammenführung (7,8) nach Art einer Strahlpumpe ausgebildet ist, bei der der zweite Teil von dem ersten Teil mitgerissen wird.

5. Vorrichtung nach Anspruch 4, **dadurch gekennzeichnet,** daß die erste Pumpe (5) in Strömungsrichtung vor dem Gasdetektor (10) liegt.

6. Vorrichtung nach Anspruch 4 oder 5, **dadurch gekennzeichnet**, daß eine zylindrische erste Kammer (3) am einen Ende einen vorzugsweise peripherisch einmündenden Gaseingang (1) mit sich daran anschließenden Leitflächen (2) und ein zentrales Gasausgangsrohr (9) aufweist, daß sich am anderen Ende der ersten Kammer (3) einerseits ein außen befindlicher Gasausgang (4) und andererseits ein zentrales Leitungsstück (7,8) befindet, wobei eine weitere Leitung, in der die erste Pumpe (5) und der Detektor (10) liegen, den Gasausgang (4) mit dem Leitungsstück (7,8) verbindet und wobei das Leitungsstück (7,8) eingangsseitig aus einem koaxialen Doppelrohrstück besteht, von dem der innere Teil mit der weiteren Leitung verbunden ist und in dem äußeren Teil des Leitungsstückes (7,8) endet und der äußere Teil sich eingangsseitig in die erste Kammer (3) öffnet und ausgangsseitig in das Gasausgangsrohr (8) übergeht.

7. Vorrichtung nach Anspruch 6, **dadurch gekennzeichnet**, daß die weitere Leitung ausgangsseitig eine zweite Kammer (6) bildet, in der sich der Gasdetektor (10) an einer Stelle befindet, wo möglichst wenig Gasbewegung herrscht.
